(19) Europäisches Patentamt
European Patent Office
Office européen des brevets

(11) EP 0 722 733 A1

(12) EUROPEAN PATENT APPLICATION
published in accordance with Art. 158(3) EPC

(43) Date of publication:
**24.07.1996 Bulletin 1996/30**

(21) Application number: **94917801.6**

(22) Date of filing: **10.06.1994**

(51) Int. Cl.[6]: **A61K 31/557**

(86) International application number:
**PCT/JP94/00949**

(87) International publication number:
**WO 95/00150 (05.01.1995 Gazette 1995/02)**

(84) Designated Contracting States:
**CH DE ES FR IT LI NL SE**

(30) Priority: **18.06.1993 JP 147324/93**

(71) Applicant: **TEIJIN LIMITED**
**Osaka-shi, Osaka 541 (JP)**

(72) Inventors:
- **SEKI, Jiro**
  **Hyogo 651 (JP)**
- **KATO, Yasuko**
  **Nishinomiya-shi Hyogo 663 (JP)**
- **NISHIO, Mie**
  **Himeji-shi Hyogo 672 (JP)**
- **WATANABE, Kunihito**
  **Tokyo 191 (JP)**

(74) Representative: **Wössner, Gottfried et al**
**Hoeger, Stellrecht & Partner,**
**Uhlandstrasse 14c**
**D-70182 Stuttgart (DE)**

# (54) BLOOD VESSEL THICKENING INHIBITOR AND SMOOTH MUSCLE FIBER MIGRATION INHIBITOR EACH CONTAINING 7-THIAPROSTAGLANDIN E 1? COMPOUND AS ACTIVE INGREDIENT

(57) A blood vessel thickening inhibitor and a smooth muscle fiber migration inhibitor each containing as the active ingredient a 7-thiaprostaglandin $E_1$ compound represented by general formula (I) and useful for inhibiting blood vessel thickening caused mainly by the migration or proliferation of vascular smooth muscle fibers after angioplasty, arterial bypass operation, and so forth, wherein $R^1$ represents hydrogen, etc.; $R^2$ represents hydrogen, etc.; $R^3$ represents linear branched alkyl, etc.; n represents 0 or 1; and symbol * represents an asymmetric carbon atom.

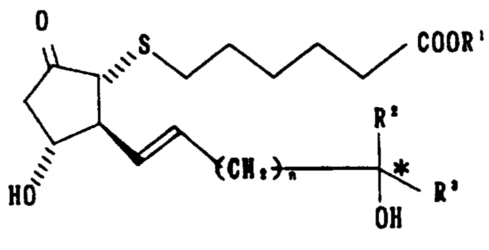

(I)

EP 0 722 733 A1

## Description

### TECHNICAL FIELD

The present invention relates to a suppressant for vascular hypertrophy and suppressant for wandering smooth muscle cells containing, as an active ingredient, a 7-thiaprostaglandin $E_1$ compound, more specifically a 7-thiaprostaglandin $E_1$ compound of the formula (I):

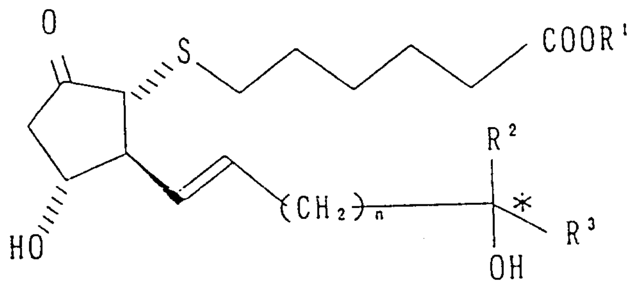

( I )

wherein $R^1$ is a hydrogen atom, a straight or branched alkyl group or one equivalent cation, $R^2$ is a hydrogen atom or methyl group, $R^3$ is a straight or branched alkyl group or cycloalkyl group, n is 0 or 1, and * is an asymmetric carbon atom.

### BACKGROUND ART

The 7-thiaprostaglandin $E_1$ compounds having the formula (I) are known compounds per se and are known to exhibit an action suppressing platelet aggregation, am action lowering the blood pressure, and a vascodilation action and result action preventing thrombus, angia, cardiac infarction, arteriosclerosis, and metastatis of malignant tumors (Japanese Unexamined Patent Publication (Kokai) No. 58-110562). The compounds are also known to have an anti-diabetic action, particularly to be useful for diabetic neuropathy (U.S. Patent No. 4937265).

On the other hand, it is known that veins frequently thicken and become occluded due to the wandering, proliferation, etc. of vascular smooth muscle cells after vascular surgery, bypass operations, and organ transplants. Various studies are under way on pharmaceuticals for the prevention and treatment of such phenomena. However, any effective pharmaceuticals have not been yet developed.

### DISCLOSURE OF THE INVENTION

Accordingly, the object of the present invention is to provide a suppressant for vascular hypertrophy and suppressant for wandering smooth muscle cells effective for the prevention and treatment of vascular hypertrophy.

In accordance with the present invention, there is provided a suppressant for vascular hypertrophy and suppressant of wandering smooth muscle cells containing at least one 7-thiaprostaglandin $E_1$ compound having the above formula (I) as an active ingredient.

### BEST MODE FOR CARRYING OUT THE INVENTION

The present inventors engaged in intensive studies under conditions conducive to the discovery of a pharmaceutical effective for the prevention and treatment of vascular hypertrophy and, as a result, found the new discovery that the compounds having the above formula (I) suppress vascular hypertrophy. They engaged in further research and completed the present invention.

The suppressant for vascular hypertrophy and the suppressant for wandering smooth muscle cells of the present invention may be used as pharmaceuticals for the prevention or treatment of vascular hypertrophy so as to suppress the thickening and occulsion of veins caused by the wandering, proliferation, etc. of vascular smooth muscle cells, which frequently occur after surgery to humans and animals.

In the above formula (I), examples of the straight or branched alkyl group shown by $R^1$, are $C_1$ to $C_{10}$ alkyl groups such as methyl, ethyl, n-propyl, iso-propyl, n-butyl, sec-butyl, tert-butyl, n-pentyl, n-hexyl, n-heptyl, n-octyl, n-nonyl, and n=decyl. Examples of the one equivalent cation are alkali metal cations such as $Na^+$ and $K^+$; bivalent or trivalent metal cations such as 1/2 $Ca^{2+}$, 1/2 $Mg^{2+}$, and 1/3 $Al^{3+}$, in particular, alkali earth metal cations and metal cations of Group 3B

of the Periodic Table; and ammonium cations such as ammonium ions and tetramethyl ammonium ions. Note that as $R^1$, a hydrogen atom or methyl group is particularly preferred.

In formula (I), $R^2$ is a hydrogen atom or methyl group, but when n is 0, a hydrogen group is particularly preferred and when n is 1, the methyl group is particularly preferred.

In formula (I), examples of the straight or branched alkyl group $R^3$ are alkyl groups having 1 to 10 carbon atoms such as ethyl, n-propyl, n-butyl, n-pentyl, n-hexyl, n-heptyl, octyl, n-decyl, 1-methylpentyl, 1-methylhexyl, 1,1-dimethylpentyl, 2-methylpentyl, 2-methylhexyl, 5-methylhexyl, 2,5-dimethylhexyl, etc., preferably n-butyl, n-pentyl, n-hexyl, (R)- or (S)- or (RS)-2-methylhexyl, particularly preferably 2-methylhexyl. Examples of the cyclohexyl groups are a $C_3$ to $C_8$ cycloalkyl group such as cyclopropyl, cyclobutyl, cyclopentyl, cyclohexyl, cycloheptyl, and cyclooctyl, particularly preferably cyclopentyl or cyclohexyl.

In formula (I), when n is 0, the compound is a 7-thiaprostaglandin $E_1$ compound where the 15-position has the same type of S-configuration as a natural configuration as shown by the following:

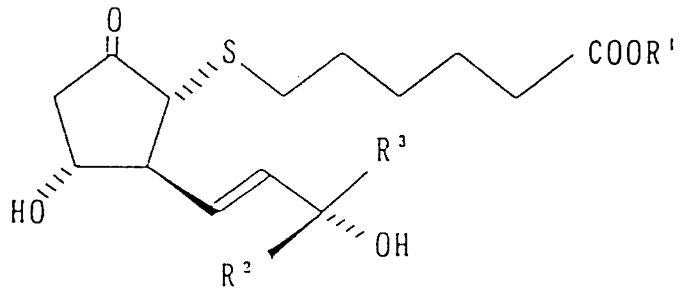

(I − 1)

where $R^1$, $R^2$, and $R^3$ are the same as defined above, and a 7-thiaprostaglandin $E_1$ compound where the 15-position has a non-natural configuration as shown by the following:

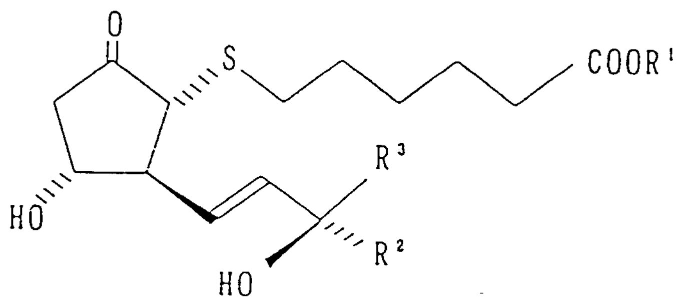

(I − 2)

where $R^1$, $R^2$, and $R^3$ are the same as defined above, or mixtures of any proportions thereof. When $R^2$ is a hydrogen atom, the 7-thiaprostaglandin $E_1$ compound having the natural configuration shown by the above formula (I-1) is particularly preferred. Even when n is 1, the 16-position R-configuration, S-configuration, and any mixtures thereof are included.

Note that the suppressant for vascular hypertrophy and suppressant for wandering smooth muscle cells of the present invention may contain either one of the above two types of stereo isomers or may contain mixtures thereof at any proportions.

Preferred specific examples of the 7-thiaprostaglandin $E_1$ compound contained in the suppressant for vascular hypertrophy and suppressant for wandering smooth muscle cells according to the present invention include the following:

(1) 7-thiaprostaglandin $E_1$
(2) 16-methyl-7-thiaprostaglandin $E_1$
(3) 20-methyl-7-thiaprostaglandin $E_1$
(4) 17,20-dimethyl-7-thiaprostaglandin $E_1$
(5) (17R)-mer of (4)
(6) (17S)-mer of (4)
(7) 15-methyl-7-thiaprostaglandin $E_1$

(8) 16,16-dimethyl-7-thiaprostaglandin $E_1$
(9) 16,17,18,19,20-pentanol-15-cyclopentyl-7-thiaprostaglandin $E_1$
(10) 16,17,18,19,20-pentanol-15-cyclohexyl-7-thiaprostaglandin $E_1$
(11) 15-deoxy-16-hydroxy-16-methyl-7-thiaprostaglandin $E_1$
(12) (16R)-mer of (11)
(13) (16S)-mer of (11)
(14) methyl esters of (1) to (13)
(15) ethyl esters of (1) to (13)
(16) tert-butyl esters of (1) to (13)
(17) sodium salts of (1) to (13)
(18) potassium salts of (1) to (13)
(19) magnesium salts of (1) to (13)
(20) ammonium salts of (1) to (13)

The 7-thiaprostaglandin $E_1$ compound (1) is produced by a known method. The production processes are described in detail in, for example, U.S. Patent Nos. 4466980 and 5159102, Japanese Unexamined Patent Publication (Kokai) No. 58-110562, or Tanaka et al "Chemical and Pharmaceutical Bulletin, vol. 33, 2359 (1985).

The suppressant for vascular hypertrophy and suppressant for wandering smooth muscle cells according to the present invention are, for example, useful as agents for the suppression for the thickening or occlusion of veins occurring mainly due to wandering or proliferation of vascular smooth muscle cells after various kinds of vascular surgery, such as surgery for formation of coronary arteries, bypass operations, organ (liver) transplants, etc. or as agents for the prevention and treatment thereof. This hypertrophy is known to be a cause of reconstriction after surgery for forming coronary arteries for example. Further, since the suppressant inhibits the wandering of vascular smooth muscle cells, it is useful as an agent for the prevention and treatment of hypertrophy, occlusion, and arteriosclerosis.

The 7-thiaprostaglandin $E_1$ compound (I) can be administered orally or non-orally such as rectally, subcutaneously, intramuscularly, intravenously, or transdermally to obtain the above object, but preferably it is administered orally or intravenously.

For the oral administration, it may be formed into a solid preparation or a liquid preparation. Examples of the solid preparation are tablets, pills, dispersions, or granules. In such solid preparations, the active ingredient is mixed with a pharmaceutically allowable carrier such as sodium bicarbonate, calcium carbonate, potato starch, sucrose, mannitol, or carboxymethylcellulose. The procedure for preparation is according to the usual method, but additives for the preparation other than the above carrier may also be added, for example, a gloss agent such as calcium stearate or magnesium stearate.

For example, it is possible to spray the above solid preparation with an organic solvent or aqueous solution of a enteric substance such as cellulose acetate phthalate, hydroxypropylmethylcellulose phthalate, polyvinylalcohol phthalate, styrene-anhydrous malate copolymer or methacrylic acid, or methacrylate copolymer to form an enteric coating and make it an enteric-coated preparation. A solid preparation such as a dispersion or granules can also be enclosed in an enteric-coated capsule.

The liquid preparation for oral administration includes, for example, an emulsifier, solubilizer, suspension agent, syrup agent, or elixer agent. These preparations include the generally pharmaceutically allowable carriers such as water or liquid paraffin. Oily substrates such as coconut oil, fractionated coconut oil, soybean oil, and corn oil may also be used as the carrier.

The pharmaceutically acceptable carriers include, other adjuvants usually used when necessary, aromatics, stabilizers, and preservatives.

Further, the liquid preparation may be administered in a capsule prepared by an absorbable substance such as gelatin.

The solid preparation for rectal administration includes a suppository including the active ingredient and prepared by a known method.

The non-orally administered preparation is administered as a sterile aqueous or nonaqueous liquid, suspension or emulsion. The nonaqueous solution or suspension may be prepared, for example, using a pharmaceutically acceptable carrier such as propyl glycol, polyethylene glycol, a vegetable oil such as olive oil, or an injectable organic ester such as ethyl oleate. These preparations may include adjuvants such as a preservative, humectant, emulsifier, dispersant, or stabilizer. These solutions, suspensions, and emulsions may be sterilized by, for example, filtering them through a bacteria retaining filter, mixing in a bactericide, or treatment by ultraviolet light etc. Further, it is possible to prepare a sterile solid preparation and dissolve it in a sterile water or sterile injection solvent just before use.

Examples of the form of the preparation which is administered, mention may be made for example of ointments. These are produced by the conventional methods.

Note that the 7-thiaprostaglandin $E_1$ compound (I) according to the present invention may be used after forming the component into an inclusive clathrate with $\alpha$-, $\beta$-, or $\gamma$-cyclodextrin or methylated cyclodextrin.

When the 7-thiaprostaglandin $E_1$ compound according to the present invention is used as a suppressant for vascular hypertrophy, the dosage differs depending on the extent of the symptoms of the patient, the age, sex, body weight, method of administration, etc., but for a normal adult about 1 μg to 1 mg may be administered per day. The dosage may be administered once a day or divided into several times a day, for example, two to six times.

EXAMPLES

The present invention will be further explained in details by the following Examples, but is by no means limited to these Examples.

Example 1: Action in Suppressing Thickening of Intima After Trauma to Rat Carotid Artery Endothelium by Balloon Catheter

Test Compound: (17R)-17,20-dimethyl-7-prostaglandin $E_1$ methyl ester

Test Method:

The test was performed in the following manner in accordance with the method of Krauz et al. (Lab. Invest., vol. 49, p. 208, 1983). Male SD rats having a body weight of 400 to 500g was used. The neck of each rat was opened under anesthesia by sodium pentobarbitol, then a balloon catheter (Fogarty, 2F) was inserted from the right external carotid artery until the starting portion of the common carotid artery. The balloon was expanded by physiological saline to such an extent causing a light resistance, then the catheter was pulled out in that state up to the carotid artery to give trauma to the intima. This procedure was repeated three times, then the catheter was withdrawn and the external carotid artery was sewn up. After 14 days, the chest was opened under anesthesia by pentobarbitol, refluxing was performed from the left ventricle by physiological saline containing heparin (20 units/ml), then the left common carotid artery was excised and immobilized by neutral formalin solution. The immobilized carotid artery was dyed by Olsein dye, then the areas of the media and thickened portion of the intima were measured by an image resolution device (LUZEX2D).

The test drug began to be administered subcutaneously at a rate of 1.0 μg/rat/hr or 3.2 μg/rat/hr using a miniosmotic pump buried in the back of the rat from two days before the surgery and was continued until 14 days after the surgery. The activity of the drug in suppressing intima thickening was found from the following formula.

$$\text{Intima thickening suppression rate (\%)} = (1\text{-}T/C) \times 100$$

wherein, T shows the ratio between the area of the thickened portion of the intima and the area of the media portion of the rats of the drug group, while C shows that of the control group (group administered solvent)

[Intima/Media].

The test results are shown in Table 1.

Table 1

| Drug | Dosage (μg/rat/hr) | No. of tests | Intima ($\mu m^2$) | Media ($\mu m^2$) | Intima/Media | Suppression rate (%) |
|---|---|---|---|---|---|---|
| Control Test Compound | 1.0 | 7 | 0.128 ±0.015 | 0.131 ±0.003 | 0.969 ±0.104 | 13.1 |
| | | 5 | 0.118 ±0.010 | 0.140 ±0.010 | 0.842 ±0.016 | |
| Control Test Compound | 3.2 | 7 | 0.161 ±0.015 | 0.176 ±0.010 | 0.918 ±0.060 | 31.4 |
| | | 8 | 0.093 ±0.013** | 0.158 ±0.008 | 0.630 ±0.100* | |
| (Average ± Standard error) | | | | | | |

*: $P < 0.05$,
**: $P < 0.01$ (comparison with control, student t-test)

As is clear from the test results shown in Table 1, the test compound suppresses the thickening of the intima of veins.

Example 2: Action in Suppressing Wandering of Vascular Smooth Muscle Cells in Rats In Vitro

Test Compound: Same as Example 1

Test Method:

Media tissue of the chest arteries of the rats was sampled in accordance with the method of Ross (J. Cell Biol. 50: 172-186, 1971), then was incubated by a Delvecco Modified Eagle Medium (DMEM) containing 10% fetal calf serum in a $CO_2$ incubator (95% air-5% $CO_2$, 37°C, humidity 100%). The fourth generation cultured cells were used for the experiment. The rat vascular smooth muscle cells (SMC) were freed by a 0.008% trypsin-0.01% EDTA solution and made to wander in DMEM containing 0.1% bovine serum albumin. The wandering of the cells was observed using a 24-well blind well chamber made by Neuro Probe. In the lower chambers were placed 100 μl amounts of a mixture of the cell wandering factor (platelet derived growth factor PDGF 20 ng/ml) and the drug. These were covered by a Nuclepore filter (pore size, 8 μm) made by Corning. 100 μl amounts of the incubation solution containing the drug and in which $5 \times 10^5$ cells/ml of SMC were made to wander were placed in the top chambers and allowed to stand in a $CO_2$ incubator for 4 hours. After the end of the incubation, the smooth muscle cells were immobilized for each filter, then were Propidum dyed. The cells in the lower chambers were counted in four fields under a laser microscope in a 0.48 $mm^2$ area field. The average was used as the number of wandering cells.

The rate of suppression of cell wandering (%) was found from the following formula.

$$\text{Cell wandering suppression rate (\%)} = 100 - (T/C \times 100)$$

wherein, C and T respectively show the number of wandering cells at the time of addition of cell wandering factor minus the number of wandering cells at the time of no addition of cell wandering factor and the number of wandering cells at the time of addition of the drug.

The test results are shown in Table 2.

Table 2

| Drug | Concentration (M) | No. of tests | No. of wandering cells (cells/0.48 $mm^2$) | Suppression rate (%) |
|---|---|---|---|---|
| Control | - | 3 | 176±19 (C) | |
| Test Compound | $10^{-9}$ | 3 | 117±19##(T) | 33.5 |
| Test Compound | $10^{-8}$ | 3 | 107±15##(T) | 39.2 |
| (Average ± Standard error) | | | | |

##: P < 0.01 (comparison with control, Dunnett t-test)

As is clear from the test results of Table 2, the test compounds suppress the wandering of vascular smooth muscle cells and, as a result, can be considered to suppress the thickening of the intima of veins.

Example 3: In Vitro Action to Suppress Migration of Vascular Smooth Muscel Cells Derived From Normal Human Arteries

Test Compound: Same as Example 1

Test Method:

To investigate the activity of the tested compound to inhibit cell migration, the migrated cells mediated by human platelet derived growth factor (PDGF) were measured in the following way using the vascular smooth muscle cells derived from normal human arteries and according to the method of MaCarthy et al. (J. Cell. Biol. 97, 772-777 (1983)). That is, a Transwell Chamber (Costar Co., registered trademark) was used to measure the inhibitory action. The chamber was divided into two layers, upper and lower, by a 8 µm pore size membrane filter (PVP - free polycarbonate filter, Nucleopore Co., registered trademark). The filter was precoated on its lower surface with 5 µg of rat tail collagen type 1 (Becton Dickinson Co.) To the upper chamber (100 µl) was added a 1.5 × $10^6$/ml suspension of cells (DMEM (made by Flow Laboratories Co.) containing 0.1% BSA (made by Nakalai Tesque Co.) In the lower chamber (600 µl) was placed the same solution diluted by human PDGF (made by Becton Dickinson) to give a final concentration of 10 ng/ml. At this time, the tested compound was added in different concentrations to both the upper and lower chambers. The chambers were incubated at 37°C under 5% $CO_2$ for 6 hours, then the filter was removed, fixed in 99.7% methanol, then stained with hematoxylin and eosin. The cells on the upper surface of the filter were removed by wiping with a cotton swab and the cells that had migrated to the lower surface of the filter were counted under a high output microscope (×200, ×400). Usually, five fields were counted per filter. The migrated cells were shown by the average cell count of five fields ± standard deviation. The significant difference was examined by the Student's two-tailed t test. The test results are shown in Table 3.

Table 3

| Drug | Concentration (M) | No. of wandering cells | Suppression rate (%) |
|---|---|---|---|
| Control | - | 86.4±20.1 | - |
| Prostaglandin $E_1$ | $10^{-9}$ | 91.0±9.8 | No significance |
| " | $10^{-8}$ | 82.4±6.9 | " |
| " | $10^{-7}$ | 82.6±17.8 | " |
| Test compound | $10^{-9}$ | 67.2±15.7* | 22.2% |
| " | $10^{-8}$ | 51.0±9.5** | 41.0% |
| " | $10^{-7}$ | 19.0±11.8** | 78.0% |

* P < 0.02
** P < 0.001

As is clear from the test results, it was found that the test compound has a special action compared with prostaglandin $E_1$.

Example 4: Acute Toxicity Test

Test Compound: (17R)-17,20-dimethyl-7-thiaprostaglandin $E_1$ methyl ester

Test Method:

The above test compound was tested for acute toxicity using SD rats (6 weeks old, SPE, male) (p.o.), whereupon the results shown in the following Table 4 were obtained.

Table 4

| Dosage (mg/kg/p.o.) | No. of deaths |
|---|---|
| 20 | 0/2 |
| 100 | 1/2 |
| 200 | 2/2 |

From these test results, the $LD_{50}$ of the test compounds was judged to be about 100 mg/kg and therefore it was found that there was no problem with acute toxicity.

Example 5

Tablets were produced with each tablet comprised of the following:

| | |
|---|---|
| Active ingredient | 200 µg |
| Lactose | 180 mg |
| Potato starch | 50 mg |
| Polyvinylpyrrolidone | 10 mg |
| Magnesium stearate | 5 mg |

The active ingredient, lactose, and potato starch were mixed, the mixture was moistened equally with a 20% ethanol solution of polyvinylpyrrolidone, then the result was passed through a 20 mm mesh sieve, dried at 45°C, and then passed once again through a 15 mm mesh sieve. The granules thus obtained were mixed with magnesium stearate and then compressed into tablets.

As the active ingredients, typically (17R)-17,20-dimethyl-7-thiaprostaglandin $E_1$ methyl ester was used.

Example 6

Hard gelatin capsules were produced with each capsule containing the following composition:

| | |
|---|---|
| Active ingredient | 100 µg |
| Microcrystalline cellulose | 195 mg |
| Amorphous silica | 5 mg |

The finely divided active ingredient, microcrystalline cellulose, and unpressed amorphous silica were sufficiently mixed and packed into hard gelatin capsules.

As the active ingredient, the same compound as in Example 5 was used.

Example 7

The same compound as in Example 5 was dissolved in fractionated coconut oil. The result was warmed and dissolved in a coating component of the following formula. A soft capsule making machine was used to prepare soft capsule agents by the usual method so as to give 100 µg of the active ingredient per capsule.

Coating Formula

| | |
|---|---|
| Gelatin | 10 parts per weight |
| Glycerol | 5 parts per weight |
| Sorbic acid | 0.08 parts per weight |
| Refined water | 14 parts per weight |

INDUSTRIAL APPLICABILITY

The suppressant for vascular hypertrophy and suppressant for wandering smooth muscle cells containing the 7-thiaprostaglandin $E_1$ compound as an active ingredient according to the present invention, as mentioned above, can be used as a drug for the suppression for wandering vascular smooth muscle cells which frequently occur after surgery to humans and animals and also as drugs for the prevention or treatment of vascular hypertrophy so as to suppress the thickening and occlusion of veins caused by such wandering, proliferation, etc.

## Claims

1. A suppressant for vascular hypertrophy containing as an active ingredient, at least one 7-thiaprostaglandin $E_1$ compound having the following formula (I):

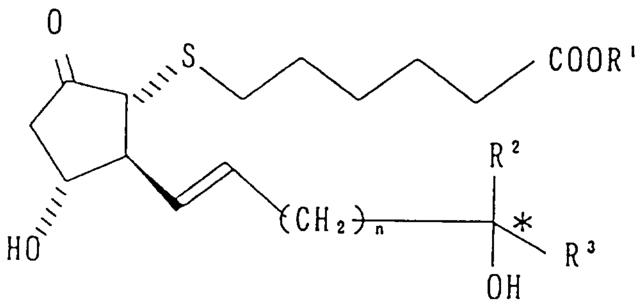

(I)

wherein $R^1$ is a hydrogen atom, a straight or branched alkyl group or one equivalent cation, $R^2$ is a hydrogen atom or methyl group, $R^3$ is a straight or branched alkyl group or cycloalkyl group, n is 0 or 1, and * is an asymmetric carbon atom.

**2.** A suppressant for vascular hypertrophy as claimed in claim 1, wherein $R^1$ is a hydrogen atom or a methyl group.

**3.** A suppressant for vascular hypertrophy as claimed in claim 1, wherein $R^2$ is a hydrogen atom.

**4.** A suppressant for vascular hypertrophy as claimed in claim 1, wherein $R^3$ is a straight or branched alkyl group having 1 to 10 carbon atoms.

**5.** A suppressant for vascular hypertrophy as claimed in claim 1, wherein $R^3$ is a 2-methylhexyl group.

**6.** A suppressant for vascular hypertrophy as claimed in claim 1, wherein the n is 0.

**7.** A suppressant for vascular hypertrophy as claimed in claim 1, wherein the absolute configuration of the asymmetric carbon atom represented by the above * is the S-configuration.

**8.** A suppressant for vascular hypertrophy as claimed in claim 1, wherein the 7-prostaglandin $E_1$ compound is (17R)-17,20-dimethyl-7-thiaprostaglandin $E_1$ methyl ester.

**9.** A suppressant for wandering smooth muscle cells containing, as an active ingredient, at least one 7-thiaprostaglandin $E_1$ compound having the following formula (I):

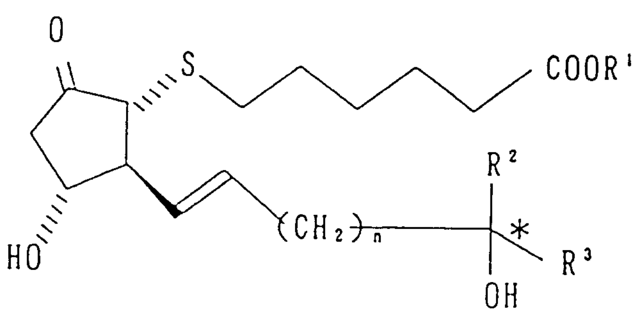

(I)

wherein $R^1$ is a hydrogen atom, a straight or branched alkyl group or one equivalent cation, $R^2$ is a hydrogen atom or methyl group, $R^3$ is a straight or branched alkyl group or cycloalkyl group, n is 0 or 1, and * is an asymmetric carbon atom.

**10.** A suppressant for wandering smooth muscle cells as claimed in claim 9, wherein $R^1$ is a hydrogen atom or a methyl group.

**11.** A suppressant for wandering smooth muscle cells as claimed in claim 9, wherein $R^2$ is a hydrogen atom.

12. A suppressant for wandering smooth muscle cells as claimed in claim 9, wherein $R^3$ is a straight or branched alkyl group having 1 to 10 carbon atoms.

13. A suppressant for wandering smooth muscle cells as claimed in claim 9, wherein $R^3$ is a 2-methylhexyl group.

14. A suppressant for wandering smooth muscle cells as claimed in claim 9, wherein the n is 0.

15. A suppressant for wandering smooth muscle cells as claimed in claim 9, wherein the absolute configuration of the asymmetric carbon atom represented by the above * is the S-configuration.

16. A suppressant for wandering smooth muscle cells as claimed in claim 9, wherein the 7-prostaglandin $E_1$ compound is (17R)-17,20-dimethyl-7-thiaprostaglandin $E_1$ methyl ester.

**INTERNATIONAL SEARCH REPORT**

International application No.
PCT/JP94/00949

**A. CLASSIFICATION OF SUBJECT MATTER**

Int. $Cl^5$ A61K31/557

According to International Patent Classification (IPC) or to both national classification and IPC

**B. FIELDS SEARCHED**

Minimum documentation searched (classification system followed by classification symbols)

Int. $Cl^5$ A61K31/557

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP, A, 58-110562 (Teijin Ltd.), July 1, 1983 (01. 07. 83), Claim, (Family: none) | 1-16 |
| A | JP, A, 61-72747 (Teijin Ltd.), April 14, 1986 (14. 04. 86), Claim & EP, A, 173754 & WO, A, 8503936 | 1-16 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| August 12, 1994 (12. 08. 94) | September 6, 1994 (06. 09. 94) |
| Name and mailing address of the ISA/ Japanese Patent Office | Authorized officer |
| Facsimile No. | Telephone No. |

Form PCT/ISA/210 (second sheet) (July 1992)